(19) Europäisches Patentamt
European Patent Office
Office européen des brevets

(11) **EP 2 928 856 B1**

(12) **EUROPEAN PATENT SPECIFICATION**

(45) Date of publication and mention
of the grant of the patent:
**11.09.2019 Bulletin 2019/37**

(51) Int Cl.:
*C07C 51/00* (2006.01)      *C07C 51/42* (2006.01)
*C07C 51/47* (2006.01)      *C07C 53/02* (2006.01)
*C07C 59/185* (2006.01)

(21) Application number: **13802053.2**

(22) Date of filing: **09.12.2013**

(86) International application number:
**PCT/EP2013/075962**

(87) International publication number:
**WO 2014/087016 (12.06.2014 Gazette 2014/24)**

(54) **PROCESS FOR THE PRODUCTION OF A BIOMASS HYDROLYSATE**

VERFAHREN ZUR HERSTELLUNG VON LÄVULINSÄURE UND/ODER AMEISENSÄURE AUS BIOMASSE

PROCÉDÉ POUR LA PRODUCTION D'ACIDE LÉVULINIQUE ET D'ACIDE FORMIQUE À PARTIR DE BIOMASSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.12.2012 EP 12196081
07.12.2012 US 201261734530 P
06.09.2013 EP 13183333**

(43) Date of publication of application:
**14.10.2015 Bulletin 2015/42**

(73) Proprietor: **Georgia-Pacific LLC
Atlanta, GA 30303 (US)**

(72) Inventors:
• **PARTON, Rudy Francois Maria Jozef
NL-6100 AA Echt (NL)**
• **KROON, Johannes Augustinus
NL-6100 AA Echt (NL)**
• **WOESTENBORGHS, Pierre Louis
NL-6100 AA Echt (NL)**

(74) Representative: **Grünecker Patent- und
Rechtsanwälte
PartG mbB
Leopoldstraße 4
80802 München (DE)**

(56) References cited:
WO-A1-2010/030617      WO-A1-2012/131665
FR-A- 1 105 538      US-A- 2 305 738
US-A- 2 648 704      US-A- 2 840 605
US-A- 6 054 611

• **RONEN WEINGARTEN ET AL: "Kinetics and
Reaction Engineering of Levulinic Acid
Production from Aqueous Glucose Solutions",
CHEMSUSCHEM, vol. 5, no. 7, 1 July 2012
(2012-07-01), pages 1280-1290, XP055061442,
ISSN: 1864-5631, DOI: 10.1002/cssc.201100717**

Remarks:
The file contains technical information submitted after the application was filed and not included in this specification

EP 2 928 856 B1

**Description**

The present invention relates to a process for the production of a biomass hydrolysate suitable for the production of levulinic acid and formic acid, to a biomass hydrolysate obtainable by said process, to a process for the production of levulinic acid and formic acid from said biomass, and to levulinic acid and formic acid obtainable by said process.

Levulinic acid is a starting molecule for the synthesis of esters known as fuel additive and is known to be useful as plasticisers and solvents. Levulinic acid can be used to synthesize methyl tetrahydrofuran (MTHF) or can be used as a solvent. Other applications of levulinic acid are for example the synthesis of delta-amino levulinic acid used as herbicides and pesticides, diphenolic acid used to synthesize polycarbonates and succinic acid used to make polyesters. Levulinic acid can also be used to produce gamma-valerolactone (5-methylbutyrolactone), which in turn can be used for production of adipic acid (1,6-hexanedioic acid).

Formic acid is used as a preservative and antibacterial agent in livestock feed in the production of leather and in dyeing and finishing of textile. It is also used as coagulant in the production of rubber as well as cleaning agent assistant and potential future fuel for fuel cells.

US8138371 relates to a process to produce formic acid from biomass. The process of US8138371 involves two subsequent hydrolysis reactions. Between the first and the second hydrolysis reaction the temperature is decreased. An example is described in Example 2 of US 8,138,371 where crude paper pulp containing 148.8 kg of cellulose is subjected to a first hydrolysis reaction in a stage tubular reactor at 205°C and at a $H2SO4$ concentration of 4 wt% and a residence time of 15 seconds. The resulting mixture is then subjected to a second hydrolysis at 185°C with a residence time of 25 minutes. This results in 33.3 kg of formic acid, corresponding to a formic acid yield of 22% which represents 82% of the theoretical yield. A yield of 62 wt% for levulinic acid is reported. US5608105 relates to a process to produce levulinic acid from biomass. Like US8138371, the process also contains two subsequent hydrolysis reactions with intermittent lowering of temperature. In Example 6 of US5608105 a 10wt% slurry of hardwood containing 5.0 wt% $H2SO4$ is subjected to a first hydrolysis reaction in a stage tubular reactor at 220°C with a residence time of 15.7 seconds. The resulting mixture is then subjected to a second hydrolysis at 210°C with a residence time of 20 minutes. The levulinic acid concentration in the liquid after the second hydrolysis is 1.05% at steady state and the yield is 62% of the theoretical yield. However, no yields and concentration of formic acid are reported. US4897497 relates to the production of levulinic acid and furfural from biomass. Like US5608105 and US8138371, the process of US4897497 contains two subsequent hydrolysis reactions with intermittent lowering of temperature. US6054611 relates to a process to produce levulinic acid from glucose or biomass where when starting from biomass two hydrolysis reactions are utilized where the acid concentration is lowered from the first to the second reaction by means of dilution.

US 2,840,605 discloses a method of making levulinic acid from lignocellulosic material, which comprises subjecting said material in comminuted form to the hydrolyzing action of a dilute sulfuric acid solution, heating the mass of ligno-cellulose material and sulfuric acid solution to a temperature of from about 140° to 210°C, holding the mass within that temperature range until at least about 15% by weight of the ligno-cellulose material on an air-dry basis has been converted into levulinic acid, cooling the hydrolysate mass including residual lignin substances to a temperature of at least about 50°C, removing tars by filtration, extracting the resulting cooled substantially tar-free hydrolysate with a ketone, immiscible with water, and recovering levulinic acid from the resulting extract.

US 2,305,738 discloses a process for the production of levulinic acid by action of diluted hydrochloric acid under pressure and at higher temperatures upon pine wood, which comprises treating the disintegrated wood with the diluted hydrochloric acid at temperatures above 100°C and at elevated pressures and so long that the conversion of the cellulose constituents of the wood takes place with avoidance of the formation of considerable quantities of simple carbohydrates directly up to the stage of the levulinic acid, the temperature being from 130° to 180°C.

FR 1105538 discloses the use of a continuous process for the preparation of levulinic acid, which process comprises the following steps: a mixture or a dispersion of a product releasing a hexose and an aqueous solution of a substantially non-volatile catalyst are continuously passed through a reaction zone which is maintained at a high temperature to obtain an important amount of levulinic acid from the hexose-releasing product. A solid residue is then separated from the reactants to give an aqueous treatment liquor containing the catalyst and levulinic acid and also some products in the dissolution state.

It is an aim of the invention to provide a simple process to produce formic acid and levulinic acid from biomass. It is another aim of the invention to provide a process to produce formic acid and levulinic acid from biomass with higher selectivity. It is another aim of the invention to provide a process to produce a biomass hydrolysate comprising formic acid and levulinic acid which is suitable for high biomass loading. It is another aim of the invention to provide a process to produce a biomass hydrolysate comprising formic acid and levulinic acid from which the formic acid and levulinic acid can be efficiently isolated.

The present invention provides a process to produce levulinic acid and formic acid, said process comprising a single hydrolysis step, said process comprising:

(a) subjecting a slurried biomass to a temperature of between 120 and 200°C for a time period of between 2 minutes and 8 hours at a mineral acid concentration of between 1-15% to yield a biomass hydrolysate; and subsequently

(b) subjecting the biomass hydrolysate to a solid-liquid separation to yield a solid fraction and a liquid fraction and recovering the liquid fraction and concentrating said liquid fraction by a vapor removal step to yield a condensate and a vapor,

or

(b') concentrating the biomass hydrolysate by a vapor removal step to yield a condensate and a vapor and subjecting the condensate to a solid-liquid separation to yield a solid fraction and a liquid fraction and recovering the liquid fraction; and subsequently

(c) subjecting the condensate obtained in step (b) or the liquid fraction obtained in step (b') to solvent-solvent extraction by adding an organic solvent to yield an organic phase comprising levulinic acid and/or formic acid and an aqueous phase; and

(d) recovering the organic phase and isolating the levulinic acid and/or the formic acid from the organic phase, wherein a condensate from the vapor obtained in step (b) or in step (b') is used to wash the solid fraction.

Preferred embodiments are set forth in the subclaims.

The claimed process includes a single hydrolysis step wherein a slurried biomass is subjected to a temperature of between 120 and 200°C, preferably between 160 and 190°C, for a time period of between 2 minutes and 8 hours, preferably between 20 and 140 minutes at a mineral acid concentration of between 1-15wt%. The process can be carried out starting from lignocellulosic biomass, and also from sugars such as glucose and fructose.

The invention provides a process to produce levulinic acid and formic acid, said process comprising a single hydrolysis step, said hydrolysis step comprising:

- subjecting a slurried biomass to a temperature of between 120 and 200°C, preferably between 160 and 190°C, for a time period of between 2 minutes and 8 hours, preferably between 20 and 140 minutes at a mineral acid concentration of between 1-15wt%.

The inventors have surprisingly found that a biomass hydrolysate comprising both levulinic acid and formic acid can be produced from biomass consisting of only one hydrolysis reaction without intermittent cooling and heating or acid dilution.

Particularly good results are obtained when the reaction is done using $H_2SO_4$ as mineral acid, and when the residence time (T, in minutes), the $H_2SO_4$ concentration ($[H_2SO_4]$, expressed in wt%), and the temperature (in K) of the reaction are such that the outcome of equation (I) lies between 0.21E-16 and 1.63E-16. Thus, the skilled person can easily, without undue burden, select suitable temperature, $H_2SO_4$ concentration, and residence time to produce a biomass hydrolysate comprising levulinic acid and formic acid.

$$T * [H_2SO_4] * \exp(-19000/T) \quad (I)$$

In other words, the temperature, residence time, and $H_2SO_4$ concentration are preferably selected such that: $0.21E\text{-}16 \geq T*[H_2SO_4]*\exp(-19000/T) \geq 1.63E\text{-}16$.

The temperature is preferably kept essentially constant during the reaction, but it may vary somewhat in the course of the reaction. This is no problem, so long as the outcome of equation I lies between 0.21E-16 and 1.63E-16. In general, the temperature may vary between +/- 15°C, preferably the variation in temperature in the course of the reaction is +/- 10°C, more preferably +/- 5°C, even more preferably +/- 3°C.

The biomass hydrolysate comprises levulinic acid and formic acid. The amount of levulinic acid in the biomass hydrolysate produced by the process of the invention is preferably at least 1.1 wt% relative to the total weight of the biomass hydrolysate, more preferably at least 1.5 wt%, even more preferably at least 2 wt%, or at least 3 wt%.

The amount of formic acid in the biomass hydrolysate produced by the process of the invention is preferably at least 0.55 wt% relative to the total weight of the biomass hydrolysate, more preferably at least 0.75 wt%, at least 1 wt%, at least 1.5 wt%.

The biomass may be or may be derived from grass, cereal, starch, algae, tree bark, hay, straw, leaves, paper pulp, paper sludge, or dung. Paper pulp, or simply pulp, is a lignocellulosic fibrous material prepared by chemically or mechanically separating cellulose from wood, fibre crops or waste paper. Pulp is rich in cellulose and other carbohydrates.

Paper sludge, or simply sludge, is a lignocellulosic fibrous containing cellulose fibres too short for usage in the paper industry. The biomass may comprise lignocellulosic biomass. Lignocellulosic biomass typically has a fibrous nature and comprises a bran fraction that contains the majority of lignocellulosic (bran) fibers. As an example, corn fiber is a heterogeneous complex of carbohydrate polymers and lignin. It is primarily composed of the outer kernel covering or seed pericarp, along with 10-25% adherent starch. Carbohydrate analyses of corn fiber vary considerably according to the source of the material. The lignocellulosic biomass may comprise hemicellulose.

In an embodiment, the biomass comprises C6 sugars, particularly fructose or glucose, or mixtures thereof. Sucrose ($C_{12}H_{22}O_{11}$) can be broken down into one molecule of glucose ($C_6H_{12}O_6$) plus one molecule of fructose (also $C_6H_{12}O_6$, an isomer of glucose), in a weakly acidic environment by a process called inversion. Fructose can also be made by enzymatic isomerization of glucose. Sucrose is commonly produced from biomass such as beet, corn and cane. When starting from sugars such as glucose or fructose, "slurried" can simply refer to a dissolved state. Thus, in such case the process comprises subjecting a solution of (dissolved) C6 sugars to a temperature of between 120 and 200°C, preferably between 160 and 190°C, for a time period of between 2 minutes and 8 hours, preferably between 20 and 140 minutes at a mineral acid concentration of between 1-15wt%.

In an embodiment, the biomass hydrolysate is made by acid hydrolysis of C6 sugars, particularly of fructose or glucose or mixtures thereof. Thus, in the context of the invention, an acid hydrolysate obtained from glucose or fructose is understood to be a biomass hydrolysate.

The mineral acid may be $H_2SO_4$, $H_3PO_4$, or HCl, or a mixture thereof. A preferred mineral acid is $H_2SO_4$. Preferred concentrations range between 3 and 14 %. Mineral acid may be added prior or during the hydrolysis step.

If the hydrolysis comprises a recycle step, the levulinic acid and formic acid concentration in the recycle stream is lower than concentration in the product stream. Preferably the recycle stream comprises an aqueous fraction of a subsequent extraction step or of a subsequent isolation step.

The hydrolysis is preferably a single pass reaction, meaning that the direct reaction product of the hydrolysis reaction is not fed back to the reactor.

The hydrolysis may be carried out in a plug-flow type reactor system. A plug-flow type reactor system results in plugflow reaction kinetics. The skilled person understands the concept of plugflow reaction kinetics and knows how to carry out the hydrolysis such that plugflow-type kinetics are obtained. Reaction kinetics are a well-known concept in the art and for example described in O. Levenspiel, "Chemical Reaction Engineering", 1998, pp 130-140.

A suitable plug-flow type reactor system is a plugflow reactor. It is possible to obtain plugflow reaction kinetics by using multiple continuous stirred tank reactor (CSTR) in series. The process may be carried out in a series of two or more, preferably three or more, four or more, more preferably five or more, even more preferably six or more batch reactors. A preferred reactor type is a tubular reactor. The tubular reactor may comprise one or more reactors. Thus, the tubular reactor may consist of two, three, four etc. tubular reactors in series. Such system can still be considered to be functionally one tubular reactor, and such process still comprises only a single hydrolysis step because the hydrolysis conditions with respect to temperature, time, and acid concentration will be substantially the same in each of the tubular reactors.

The concentration of the biomass in the slurried biomass may range anywhere between 1 and 75 wt%. A preferred concentration is between 10 and 50 wt%, more preferably between 15 and 50 wt%, all based on total weight of the slurried biomass. The slurried biomass may have a concentration of between 25-50 wt%, 30-50 wt%, 35-50 wt%, 40-50 wt%, or even 45-50 wt%. Using high biomass concentrations may result in a biomass hydrolysate having higher contents (wt%) of formic acid and/or levulinic acid. This has the additional advantage that any subsequent process steps such as the isolation of formic acid and/or levulinic acid e.g. including filtration, distillation, and/or solvent-solvent extraction can be carried out at smaller volumes meaning smaller equipment and concomitant lower investment cost and a lower environmental impact.

The wood concentrations used in biomass hydrolysis as described in the Examples of US8138371 are 10 wt% or less. The inventors have tried to use higher wood concentrations up 20 wt% and higher but found the process of US8138371 not suitable for such high concentrations as it led to clogging of their first hydrolysis reactor. The prescribed hydrolysis times in US8138371 range between 10 and 60 seconds; in the Examples of US8138371, the first hydrolysis reaction is typically 20 seconds or less. Instead, the process of the invention uses only a single hydrolysis step, which step is longer than 60 seconds and which does not result in any substantial clogging even at high biomass concentrations and which is very suitable for using high biomass loading.

The process may further comprise, prior to the hydrolysis step, an impregnation step to form a biomass slurry. The conditions of the impregnation step are not critical. In another aspect the present specification discloses a biomass hydrolysate obtainable by the process of the invention. Said biomass hydrolysate may have several advantages. The biomass hydrolysate produced by the process of the invention may further comprise char. Tar and char represent organic material which is insoluble in water, which is dark in colour and which tends to become viscous and very dark to almost black when concentrated. Tar can be formed during heating of organic material, for example by pyrolysis, but is also formed when carbohydrates are subjected to acid hydrolysis, particularly when done at high temperatures. Char usually refers to solid material, for example the remains of solid biomass that has been incompletely combusted, such as charcoal if wood is incompletely burned. Tar usually refers (viscous) liquid, e.g. derived from the destructive distillation of organic matter. Char may negatively affect the isolation of a bio-based product e.g. because of its stickiness. In the context of the invention, "char" is understood to include tar.

The process of the invention is advantageous in that any char which is produced does not hamper subsequent isolation of formic acid and levulinic acid from the biomass hydrolysate, or to a lesser extent, as compared to biomass hydrolysates produced by processes known in the art. Also, due to the favourable properties of any char produced by the process, the char does not negatively affect the hydrolysis reaction as much, for example it results in little of no clogging of the reactor.

The present specification also discloses char obtainable by the process of the invention. This char may be isolated from the biomass hydrolysate, for example by

extraction, membrane filtration, or solid-liquid separation or a combination thereof. This char is non-sticky and has a favourable particle size which

makes it easily to handle.

The formic acid and the levulinic acid can be recovered from the biomass hydrolysate by methods known in the art, such as extraction and distillation, see for example US2010/0324310.

In the claimed process, a vapor condensate is used to wash the solid

fraction. Formic acid in the condensate may thus not be lost but is retained. Any levulinic acid bound to the solid fraction (e.g. bound to char) may also be washed therefrom and thus retained. Also, this step may reduce water consumption as no or little external water is required.

The present specification further discloses formic acid obtainable by the process of the invention.

The formic acid may be in the form of a

composition, preferably an aqueous composition.

The present specification further discloses levulinic acid obtainable by the

process of the invention. The formic acid may be in the form of a

composition, preferably an aqueous composition.

## EXAMPLES

### Example 1

10ml microwave tubes were prepared as follows: a stirring bar was added and 700 mg of soft wood biomass (ground and sieved; particle size <1mm) was added. Next the biomass was impregnated. For the acid hydrolysis reaction, the tubes were capped and placed in the carrousel of a microwave and the microwave programmed as follows: 1 minute pre-stirring, 190°C, 1 minute; 1 minute pre-stirring, 190°C, 2 minutes; 1 minute pre-stirring, 190°C, 5 minutes; 1 minute pre-stirring, 190°C, 10 minutes. The acid hydrolysis was done in the presence of approximately 5 wt% hydrosulphuric acid. After the acid hydrolysis and cooling down, aliquots of the resulting biomass hydrolysate were taken from the supernatant and analyzed with HPLC. Results of the resulting composition are in Table 1.

**Table 1.**

|  | Yield (as % of theoretical yield) | Content (wt%) |
|---|---|---|
| Levulinic acid | 53.9 | 1.9 |
| Formic acid | 61.8 | 0.9 |

### Example 2

100g wood chips were impregnated for 90 minutes. After impregnation, the temperature was raised to the reaction temperature and the slurry was subjected to acid hydrolysis in the presence of approximately 5 wt% hydrosulphuric acid without stirring. The resulting biomass hydrolysate suspension was subjected to solid/liquid separation. Results of the liquid fraction and the reaction conditions are stated in Table 2.

**Table 2**

| No | time in min | T in °C | $H_2SO_4$ in wt%* | LA in wt% | FA in wt% | yield LA in % | yield FA in % |
|---|---|---|---|---|---|---|---|
| 1 | 90 | 170 | 4 | 3.952 | 1.940 | 42.4 | 51.4 |

(continued)

| No | time in min | T in °C | $H_2SO_4$ in wt%* | LA in wt% | FA in wt% | yield LA in % | yield FA in % |
|----|-------------|---------|-------------------|-----------|-----------|---------------|---------------|
| 2 | 240 | 160 | 4 | 4.396 | 2.041 | 46.3 | 53.1 |
| 3 | 180 | 170 | 2 | 4.298 | 2.037 | 47.7 | 53.6 |
| * concentration on total mass (liquor + wood) | | | | | | | |

**Example 3**

The liquid fraction of the biomass hydrolysate of Example 2 can be cooled via evaporation resulting in a vapor. The resulting vapor can be condensed resulting in an aqueous solution of 1% formic acid, 0.02% acetic acid and 0.02 % levulinic acid.

**Comparative Example A**

365g of the biomass hydrolysate suspension was filtered over a filter cloth with a pressure difference of 0.1bar. The filter cake was washed three times with 50g water. The conductivity of the wash water, an indication for the ion content (organic acids and sulfuric acid) was measured to be 225.2 mS/cm in the first filtrate, 30.02 mS/cm in the first wash, 3.52 mS/cm in the second wash and 0.786 mS/cm in the third.

**Example 4**

A biomass hydrolysate was enriched with levulinic acid to a levulinic acid concentration of 9.07wt% and with formic acid to a formic acid concentration of 1.89wt% to simulate the flash step in Example 3. 2.1 kg reaction solution was 5 times extracted 1.7kg of fresh methyltetrahydrofuran at 60°C. After the fifth extraction 99.1 % of the levulinic acid and 98.8% of the formic acid present in the reaction solution could be collected in the organic layer.

**Claims**

1. Process to produce levulinic acid and formic acid, said process comprising a single hydrolysis step, said process comprising:

   (a) subjecting a slurried biomass to a temperature of between 120 and 200°C for a time period of between 2 minutes and 8 hours at a mineral acid concentration of between 1-15% to yield a biomass hydrolysate; and subsequently
   (b) subjecting the biomass hydrolysate to a solid-liquid separation to yield a solid fraction and a liquid fraction and recovering the liquid fraction and concentrating said liquid fraction by a vapor removal step to yield a condensate and a vapor,
   or
   (b') concentrating the biomass hydrolysate by a vapor removal step to yield a condensate and a vapor and subjecting the condensate to a solid-liquid separation to yield a solid fraction and a liquid fraction and recovering the liquid fraction; and subsequently
   (c) subjecting the condensate obtained in step (b) or the liquid fraction obtained in step (b') to solvent-solvent extraction by adding an organic solvent to yield an organic phase comprising levulinic acid and/or formic acid and an aqueous phase; and
   (d) recovering the organic phase and isolating the levulinic acid and/or the formic acid from the organic phase, wherein a condensate from the vapor obtained in step (b) or in step (b') is used to wash the solid fraction.

2. Process according to claim 1, wherein the biomass is a lignocellulosic biomass.

3. Process according to claim 1, wherein the biomass comprises glucose or fructose or a combination thereof.

4. Process according to any one of claims 1 to 3, wherein the hydrolysis is carried out in a plug-flow type reactor system.

5. Process according to any one of claims 1 to 4, wherein the concentration of the biomass in the slurried biomass is between 15 and 50 wt% based on total weight of the slurried biomass.

6. Process according to any one of claims 1 to 5, further comprising, prior to the hydrolysis step, an impregnation step.

7. Process according to any one of claims 1 to 6, wherein the levulinic acid and/or the formic acid is isolated from the organic phase by distillation.

8. Process according to any one of claims 1 to 7, wherein the concentration of the biomass in the slurried biomass is between 40 and 50 wt% based on total weight of the slurried biomass.

**Patentansprüche**

1. Verfahren zur Herstellung von Lävulinsäure und Ameisensäure, wobei das Verfahren einen einzigen Hydrolyseschritt umfasst, wobei das Verfahren umfasst, dass:

(a) eine aufgeschlämmte Biomasse über einen Zeitraum zwischen 2 Minuten und 8 Stunden bei einer Mineralsäurekonzentration zwischen 1-15 % einer Temperatur zwischen 120 und 200 °C unterzogen wird, um ein Biomassenhydrolysat zu ergeben; und anschließend
(b) das Biomassenhydrolysat einer Fest-Flüssig-Trennung unterzogen wird, um eine feste Fraktion und eine flüssige Fraktion zu ergeben, und die flüssige Fraktion zurückgewonnen wird und die flüssige Fraktion durch einen Dampfentfernungsschritt eingeengt wird, um ein Kondensat und einen Dampf zu ergeben,
oder
(b') das Biomassenhydrolysat durch einen Dampfentfernungsschritt eingeengt wird, um ein Kondensat und einen Dampf zu ergeben, und das Kondensat einer Fest-Flüssig-Trennung unterzogen wird, um eine feste Fraktion und eine flüssige Fraktion zu ergeben, und die flüssige Fraktion zurückgewonnen wird; und anschließend
(c) das in Schritt (b) erhaltene Kondensat oder die in Schritt (b') erhaltene flüssige Fraktion einer Lösungsmittel-Lösungsmittel-Extraktion durch Zugeben eines organischen Lösungsmittels unterzogen wird, um eine organische Phase, die Lävulinsäure und/oder Ameisensäure umfasst, und eine wässrige Phase zu ergeben; und
(d) die organische Phase zurückgewonnen wird und die Lävulinsäure und/oder die Ameisensäure aus der organischen Phase isoliert wird,
wobei ein Kondensat aus dem in Schritt (b) oder in Schritt (b') erhaltenen Dampf verwendet wird, um die feste Fraktion zu waschen.

2. Verfahren nach Anspruch 1, wobei die Biomasse eine Lignocellulose-Biomasse ist.

3. Verfahren nach Anspruch 1, wobei die Biomasse Glucose oder Fructose oder eine Kombination davon umfasst.

4. Verfahren nach einem der Ansprüche 1 bis 3, wobei die Hydrolyse in einem pfropfenstromartigen Reaktorsystem durchgeführt wird.

5. Verfahren nach einem der Ansprüche 1 bis 4, wobei die Konzentration der Biomasse in der aufgeschlämmten Biomasse zwischen 15 und 50 Gew.-%, bezogen auf das Gesamtgewicht der aufgeschlämmten Biomasse, liegt.

6. Verfahren nach einem der Ansprüche 1 bis 5, ferner umfassend einen Imprägnierungsschritt vor dem Hydrolyseschritt.

7. Verfahren nach einem der Ansprüche 1 bis 6, wobei die Lävulinsäure und/oder die Ameisensäure durch Destillation aus der organischen Phase isoliert wird.

8. Verfahren nach einem der Ansprüche 1 bis 7, wobei die Konzentration der Biomasse in der aufgeschlämmten Biomasse zwischen 40 und 50 Gew.-%, bezogen auf das Gesamtgewicht der aufgeschlämmten Biomasse, liegt.

**Revendications**

1. Procédé pour produire de l'acide lévulinique et de l'acide formique, ledit procédé comprenant une unique étape d'hydrolyse, ledit procédé comprenant :

(a) la soumission d'une biomasse en suspension épaisse à une température comprise entre 120 et 200 °C pendant une période de temps comprise entre 2 minutes et 8 heures à une concentration en acide minéral comprise entre 1 et 15 % pour donner un hydrolysat de biomasse ; et par la suite
(b) la soumission de l'hydrolysat de biomasse à une séparation solide-liquide pour donner une fraction solide et une fraction liquide et la récupération de la fraction liquide et la concentration de ladite fraction liquide par une étape d'élimination à la vapeur pour donner un condensat et une vapeur,
ou
(b') la concentration de l'hydrolysat de biomasse par une étape d'élimination à la vapeur pour donner un condensat et une vapeur et la soumission du condensat à une séparation solide-liquide pour donner une fraction solide et une fraction liquide et la récupération de la fraction liquide ; et par la suite
(c) la soumission du condensat obtenu à l'étape (b) ou de la fraction liquide obtenue à l'étape (b') à une extraction solvant-solvant en ajoutant un solvant organique pour donner une phase organique comprenant de l'acide lévulinique et/ou de l'acide formique et une phase aqueuse ; et
(d) la récupération de la phase organique et l'isolement de l'acide lévulinique et/ou de l'acide formique par rapport à la phase organique,
dans lequel un condensat provenant de la vapeur obtenue à l'étape (b) ou à l'étape (b') est utilisé pour laver la fraction solide.

2. Procédé selon la revendication 1, dans lequel la biomasse est une biomasse lignocellulosique.

3. Procédé selon la revendication 1, dans lequel la biomasse comprend du glucose ou du fructose ou une combinaison de ceux-ci.

4. Procédé selon l'une quelconque des revendications 1 à 3, dans lequel l'hydrolyse est effectuée dans un système de réacteur de type écoulement piston.

5. Procédé selon l'une quelconque des revendications 1 à 4, dans lequel la concentration de la biomasse dans la biomasse en suspension épaisse est comprise entre 15 et 50 % en poids sur la base du poids total de la biomasse en suspension épaisse.

6. Procédé selon l'une quelconque des revendications 1 à 5, comprenant en outre, avant l'étape d'hydrolyse, une étape d'imprégnation.

7. Procédé selon l'une quelconque des revendications 1 à 6, dans lequel l'acide lévulinique et/ou l'acide formique sont isolés à partir de la phase organique par distillation.

8. Procédé selon l'une quelconque des revendications 1 à 7, dans lequel la concentration de la biomasse dans la biomasse en suspension épaisse est comprise entre 40 et 50 % en poids sur la base du poids total de la biomasse en suspension épaisse.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8138371 B
- US 5608105 A
- US 4897497 A
- US 6054611 A
- US 2840605 A
- US 2305738 A
- FR 1105538
- US 20100324310 A

**Non-patent literature cited in the description**

- **O. LEVENSPIEL.** *Chemical Reaction Engineering,* 1998, 130-140